# EUROPÄISCHE PATENTSCHRIFT

(11) **EP 1 601 456 B1**
(45) Veröffentlichungstag und Bekanntmachung des Hinweises auf die Patenterteilung: **30.09.2009**
(21) Anmeldenummer: 04713043.0
(22) Anmeldetag: 20.02.2004
(51) Int. Cl.: B01J 19/24

(54) **VERFAHREN ZUR HERSTELLUNG VON POLYISOCYANATEN**
METHOD FOR PRODUCING POLYISOCYANATES
PROCEDE DE PRODUCTION DE POLYISOCYANATES

(30) Priorität: 11.03.2003 DE 10310888
(43) Veröffentlichungstag der Anmeldung: 07.12.2005
(73) Patentinhaber: BASF SE, 67056 Ludwigshafen (DE)
(72) Erfinder: BRODHAGEN, Andreas, 67125 Dannstadt-Schauernheim (DE); SOHN, Martin, 35102 Lohra (DE); NEVEJANS, Filip, B-9170 St.Gillis-Waas (BE); STROEFER, Eckhard, 68163 Mannheim (DE); WÖLFERT, Andreas, 74906 Bad Rappenau (DE); OEHLENSCHLÄGER, Steffen, 67063 Ludwigschafen (DE)
(86) Internationale Anmeldenummer: PCT/EP2004/001673
(87) Internationale Veröffentlichungsnummer: WO 2004/080587

(56) Entgegenhaltungen:
- DE-A- 1 595 772
- US-A- 4 419 295
- US-A- 5 779 994

## Beschreibung

Die vorliegende Erfindung betrifft ein neues Verfahren zur Herstellung von Isocyanaten durch Umsetzung von primären Aminen mit Phosgen.

Polyisocyanate werden in großen Mengen hergestellt und dienen hauptsächlich als Ausgangsstoffe zur Herstellung von Polyurethanen. Ihre Herstellung erfolgt zumeist durch Umsetzung der entsprechenden Amine mit Phosgen.

In der Regel ist die kontinuierliche Ausführungsform dieses Verfahrens zweistufig. In der ersten Stufe der Phosgenierung wird das Amin mit Phosgen zu Carbamoylchlorid und Chlorwasserstoff und in einer parallelen Reaktion zu Aminhydrochlorid umgesetzt. Die Reaktion zwischen Amin und Phosgen ist sehr schnell, stark exotherm und läuft schon bei sehr niedrigen Temperaturen ab. Um Nebenprodukte- und Feststoffbildung zu minimieren, müssen daher Amin und Phosgen, gegebenenfalls in Mischung mit organischem Lösungsmittel, schnell vermischt werden. Daher erfolgt die erste Phosgenierstufe in der Regel in einem Mischorgan, vorzugsweise einer Düse. Die zweite Stufe der Phosgenierung umfasst sowohl die Zersetzung des Carbamoylchlorids zum gewünschten Isocyanat und Chlorwasserstoff als auch die Phosgenierung des Aminhydrochlorids zum Carbamoylchlorid. Die Temperatur der zweiten Phosgenierstufe ist in der Regel höheren als die der ersten. Für die zweite Stufe sind eine Vielzahl von Reaktoren entwickelt worden.

Es ist bekannt, dass durch eine Einengung der Verweilzeitverteilung verbesserte Ergebnisse der Phosgenierung bei gleicher Verweilzeit erzielt werden. Daher sind in der Literatur bereits verschiedene Verfahren zur Herstellung von Isocyanaten durch Umsetzung von Aminen mit Phosgen in der Flüssigphase unter Einhaltung einer engen Verweilzeitverteilung beschrieben. Eine enge Verweilzeitverteilung kann beispielsweise durch Verwendung einer Kaskade von Rührkesseln oder durch Verwendung eines Rohrreaktors erzielt werden.

Die einfachste Möglichkeit der Verwendung einer Kaskade als Apparatekombination mit Rohrreaktorverweilzeitcharakteristik ist die Phosgenierung in einer Rührkesselkaskade, wie in DE 844 896 beschrieben. Nachteilig ist wobei hier die Verwendung bewegter Teile. Bei phosgenführenden Apparaten sollte aus Sicherheitsgründen, insbesondere wegen der Gefahr von Undichtigkeiten an Wellendurchführungen, auf die Verwendung von bewegten Teilen in Apparaten verzichtet werden.

EP 322,647 beschreibt einen Phosgenierturm mit Lochböden als Einbauten zur kontinuierlichen Herstellung von Mono- oder Polyisocyanaten. Durch die Löchböden als Einbauten soll eine Kaskadierung der Strömung erreicht werden, was zu einer engeren Verweilzeitverteilung führt. Nachteilig ist die Verstopfungsneigung der Lochböden im Dauerbetrieb.

EP 716,079 beschreibt die Umsetzung von Amin und Phosgen in einer Blasensäule oder einem Schlaufenreaktor als Rohrreaktor mit einem nachgeschaltenem Reaktor zur Umsetzung von Amin-Hydrochlorid und einer Rückführung der Reaktionsmischung. Nachteilig ist bei diesem Verfahren, dass sich wegen der hohen zurückgeführten Mengen eine geringe Raumzeitausbeute ergibt.

DE 2,747,524 beschreibt die Phosgenierung in einem Rohrreaktor, wobei die Rohrwand insbesondere im Bereich nach der Einmischung von Amin und Phosgen so beheizt wird, dass sich das entstehende Carbamoylchlorid nicht an der Rohrwand anlagern soll.

DE 2,112,181 beschreibt die Phosgenierung in einem mit Füllkörpern gefüllten Rohrreaktor im Gleichstrom, wobei die Strömung im Rohrreaktor im Übergangsregime zwischen laminarer und pulsierender Strömung gehalten wird, und wobei ein Teil der fertigen Reaktionslösung wieder in den Rohrreaktor zurückgeführt wird. Nachteilig ist die Verstopfungsneigung des Reaktors durch Anlagerung von Feststoffen an den Füllkörpern.

DE 2,058,032 beschreibt eine zweistufige Phosgenierung, bei der die Heißphosgenierung in einem waagrechten Rohr mit eingebautem Rührer, beispielsweise Wendelrührer oder Förderschnecke, mit seitlichen Gasabzugsmöglichkeiten durchgeführt wird. Nachteilig ist auch hier die Verwendung bewegter Teile.

DE 949,228 beschreibt ein Verfahren zur kontinuierlichen Herstellung von aromatischen einkernigen Diisocyanaten. Das kontinuierliche Verfahren zur Herstellung von Isocyanaten aus primären Aminen mit Phosgen ist **dadurch gekennzeichnet, dass** in einer zweistufigen Kalt- Heißphosgenierung die Heißphosgenierung in einem senkrechten oder schrägstehenden Rohr bzw. Turm unter Zusatz von gasförmigen Phosgen durchgeführt wird. Nachteilig ist jedoch die schlechte Raumzeitausbeute der Kalt-Heißphosgenierung.

DE 3,121,036 beschreibt ein kontinuierliches Verfahren zur Herstellung von Isocyanaten, gekennzeichnet durch einstufige Temperaturführung in einer Kombination von Mischdüse und Rohrreaktor. Nachteilig bei dem Verfahren ist, dass durch den Verzicht auf eine Zwischenentgasung das Volumen des Rohrreaktors sehr groß ist.

US 3,544,612 beschreibt ein Verfahren, bei dem im Gegenstrom in einem Rohrreaktor bzw. einer Kolonne Isocyanat durch die Phosgenierung von Aminhydrochlorid hergestellt wird, wobei die Hydrochlorid-Dispersion am Kopf und die Phosgenlösung im Sumpf zugegeben wird. Zur Intensivierung des Stoffaustausches zwischen Phosgenlösung und Hydrochlorid-Dispersion werden inerten Packungen bzw. Platten empfohlen. Nachteilig bei dem Verfahren ist vor allem die Verstopfungsneigung der Einbauten und die lange Phosgenierzeit des Aminhydrochlorides.

DE 1,593,412 beschreibt ein Verfahren zur Herstellung von Isocyanaten bei erhöhten Drücken in einer Destillationskolonne. Die Durchführung des Verfahrens findet bevorzugt in einer Destillationskolonne statt, wobei der Feed aus der Kaltphosgenierung im oberen Drittel der Kolonne zugegeben wird. Am Kopf der Kolonne fallen Chlorwasserstoff und teilweise Phosgen an. Am Sumpf wird die Rohisocynatlösung abgezogen. Als Kolonneneinsätze werden Füllkörper (Raschig-Ringe) empfohlen. Nachteilig an dem Verfahren ist der rückvermischte Sumpfumlauf und die Verstopfungsneigung in den Füllkörpern. Bei der Ausführung des Verfahrens als Bodenkolonne hat man noch den Nachteil, dass man große ungenützte Gasräume hat, die das Verfahren wenig kompakt werden lassen.

US 3829458 beschreibt einen im Gleichstrom durchströmten Rohr-Reaktor mit Packungen zur Umsetzung von Amin und Phosgen, wobei die Strömung im Reaktor im Übergangszustand zwischen laminar und turbulent ist. Nachteilig ist, dass die Verwendung von Packungen im Phosgenierungsreaktor übermäßig feststoffanfällig ist.

DD 300 168 beschreibt einen horizontalen Rohrreaktor mit Eindüsung von Amin und Phosgen, wobei das Phosgen ober- und unterhalb des Amins eingedüst wird. Nachteilig ist, dass sich wegen des einfachen horizontalen Aufbaus ohne weitere Maßnahmen ein hoher Gasanteil im Rohr und eine relative breite Verweilzeitverteilung ergibt. Dies ergibt eine relativ schlechte Raumzeitausbeute und damit eine wenig kompakte Bauweise.

Aufgabe der Erfindung war es, ein Verfahren zur Herstellung von Isocyanaten bereitzustellen, welches es gestattet, die resultierende(n) Reaktion(en) unter hoher Selektivität und hoher Raum-Zeit-Ausbeute durchzuführen, so dass das Verfahren räumlich kompakt aufgebaut werden kann, und mit hoher Betriebssicherheit bezüglich Verstopfungen betrieben werden kann.

Die Aufgabe konnte gelöst werden durch ein Verfahren zur Herstellung von Polyisocyanaten durch Umsetzung von primären Aminen mit Phosgen, umfassend die Schritte
a) Mischung des Amins mir dem Phosgen,
b) Umsetzung des Amins mit dem Phosgen in einem Verweilzeitreaktor, gegebenenfalls
c) Überführung des Austrags des Reaktors aus Schritt b) in eine Destillationskolonne, **dadurch gekennzeichnet, dass** der Verweilzeitreaktor in Schritt b) als Rohr-reaktor ausgestaltet ist, gemäß Anspruch 1.

Zur Ausgestaltung des Rohrreaktors gibt es prinzipiell 4 Möglichkeiten.
1. Der Rohrreaktor steht vorwiegend senkrecht und wird von unten nach oben durchströmt. Der Vorteil dieser Ausgestaltung ist der Gleichstrom von Blasen und Flüssigkeit und die relativ stabile Betriebsweise ohne Flüssigkeitsstau und Spucken. Nachteilig ist, dass die Strömung eine gewisse Intensität haben muss, damit die bei der Phosgenierung auftretenden Feststoffe nicht sedimentieren,. Dies ergibt einen relativ engen Betriebsbereich für den bestimmungsgemäßen Betrieb des Rohrreaktors. Beispielsweise wäre bei Teillast die Turbulenzintensität zu gering. Die Feststoffe würden sedimentieren, und der Reaktor würde verstopfen.
2. Der Rohrreaktor steht vorwiegend senkrecht und wird von oben nach unten durchströmt. Der Vorteil dieser Ausgestaltung ist, dass die Feststoffe nicht sedimentieren können. Nachteilig ist, dass Gas und Flüssigkeit im Gegenstrom geführt werden. Dadurch kann es auftreten, dass der Flüssigkeitsstrom durch zusammenhängende Großgasblasen gestaut wird. Der Apparat arbeitet dann pulsierend, was eine instabile, nicht erwünschte Betriebsweise darstellt.
3. Durch Wahl eines engen Rohrdurchmessers wird eine homogene Strömung erzeugt. Dadurch wird die Geschwindigkeit von Flüssigkeit und Gas soweit gesteigert, dass die Strömung homogenisiert wird. Gas und Flüssigkeit strömen gleichmäßig über den Querschnitt verteilt ohne Ausbildung einer Schichtenstruktur oder von Schwällen. Unter dem Begriff "Schwall" wird eine Welle der Flüssigkeit verstanden, bei der in einem zweiphasigen System die Flüssigkeit den gesamten Querschnitt des Rohres einnimmt. Dadurch wird die Gasphase im Reaktor geteilt. Der Vorteil dieser Ausgestaltung ist, dass es zu keiner Sedimentation von Feststoff kommt. Nachteilig ist, dass aufgrund der hohen Strömungsgeschwindigkeit sehr große Rohrlängen erforderlich sind, um die gewünschte Verweilzeit zu erreichen. Darüber hinaus steigt mit der Rohrlänge der Druckverlust linear und sogar quadratisch mit der an sich gewünschten Vergrößerung der Strömungsgeschwindigkeit. Kommt es trotz der hohen Geschwindigkeit zu Ablagerungen an der Wand, ist eine Reinigung des engen Rohres im Vergleich zu einem größeren Querschnitt schwieriger.
4. Der Rohrreaktor ist vorwiegend horizontal. Vorteil dieser Ausgestaltung ist, dass das Sedimentieren von Feststoffen wird bereits bei sehr geringen Strömungsgeschwindigkeiten verhindert wird. Nachteilig ist, dass eine instabile Phasentrennung auftreten kann, was insbesondere bei Umlenkungen zur Entstehung von Schwällen in der Flüssigkeit führen kann. Durch Schwälle, welche sich mit einem Mehrfachen der mittleren Strömungsgeschwindigkeit bewegen, werden erhebliche mechanische Belastungen des Apparates verursacht. Dies kann im Dauerbetrieb zu einer Schädigung des Reaktors sowie daran angeschlossener Komponenten und in der Folge zum Austritt von Produkt in die Umgebung führen. Darüber hinaus wird aufgrund der hohen Geschwindigkeit der Schwälle im Vergleich zur mittleren Strömungsgeschwindigkeit die Verweilzeitverteilung verbreitert. Dies führt nach den aktuellen Erkenntnissen, die zur vorliegenden Erfindung geführt haben, zur Herabsetzung der Produktqualität. Da die Gasphase durch die Schwälle am Vorauseilen gehindert wird, stellt sich im Bereich der Schwallströmung ein geringer Schlupf und somit ein hoher Gasvolumenanteil ein. Dies führt zu einer wenig kompakten und damit teuren Bauweise.

Somit bestand eine weitere Aufgabe der Erfindung darin, den Rohrreaktor so auszugestalten, dass eine gleichmäßige Verweilzeitverteilung des Reaktionsgemisches erreicht wird und das Auftreten von Schwällen im Reaktor verhindert werden kann.

Diese Aufgabe konnte überraschenderweise dadurch gelöst werden, dass der Rohrreaktor aus einem oder mehreren horizontalen und/oder vertikalen Segmenten besteht, die untereinander durch Abschnitte verbunden sind, wobei die verbindenden Abschnitte einen Durchmesser aufweisen, der geringer ist als der Durchmesser des engsten horizontalen oder vertikalen Segments.

Gegenstand der Erfindung ist daher ein Verfahren zur Herstellung von Polyisocyanaten durch Umsetzung von primären Aminen mit Phosgen, umfassend die Schritte
a) Mischung des Amins mir dem Phosgen,
b) Umsetzung des Amins mit dem Phosgen in einem Verweilzeitreaktor, gegebenenfalls
c) Überführung des Austrags des Reaktors aus Schritt b) in eine Destillationskolonne, **dadurch gekennzeichnet, dass** der Rohrreaktor in Schritt b) aus einem oder mehreren horizontalen und/oder vertikalen Segmenten besteht, die untereinander durch Abschnitte verbunden sind, wobei die verbindenden Abschnitte einen Durchmesser aufweisen, der geringer ist als der Durchmesser des engsten horizontalen oder vertikalen Segments.

Gegenstand der Erfindung ist weiterhin die Verwendung eines Rohrreaktors, der aus einem oder mehreren horizontalen und/oder vertikalen Segmenten besteht, die untereinander durch Abschnitte verbunden sind, wobei die verbindenden Abschnitte einen Durchmesser aufweisen, der geringer ist als der Durchmesser des engsten horizontalen oder vertikalen Segments, zur Herstellung von Polyisocyanaten durch Umsetzung von primären Aminen mit Phosgen.

Der Rohrreaktor hat üblicherweise keine Einbauten.

Der Rohrreaktor kann in einer Ausführungsform der Erfindung in linearer Bauweise ausgestaltet werden, das heißt er besteht aus mehreren horizontalen Segmenten, die durch horizontale verbindende Abschnitte miteinander verbunden sind. Diese Ausgestaltung ist sehr einfach, nachteilig ist jedoch, dass bei der Verwendung einer großen Zahl von horizontalen Segmenten eine sehr große Länge des Rohrreaktors resultiert, was zu einer wenig kompakten Ausgestaltung der Anlage führt.

In einer bevorzugten Ausführungsform der Erfindung werden daher die verbindenden Abschnitte als Rohrleitungskrümmer ausgestaltet. Der Reaktor kann in dieser Ausführungsform aus mehreren vorwiegend horizontal verlaufenden Segmenten bestehen.

In einer weiteren bevorzugten Ausführungsform des Rohrreaktors werden vorwiegend horizontale Abschnitte mit vorwiegend vertikalen Abschnitten kombiniert, um beispielsweise die Aufgaben der Reaktion und der Überwindung einer räumlichen Distanz zwischen den Apparaten für die Reaktionsschritte a) und c) miteinander zu verknüpfen. Die einzelnen Segmente sind an ihren Enden über Rohrleitungskrümmer verbunden. Die Segmente können dabei neben- und übereinander angeordnet sein, um eine besonders kompakte Bauweise zu erreichen.

Dadurch, dass der Durchmesser der verbindenden Abschnitte geringer gewählt wird als der Durchmesser der durch sie zu verbindenden Rohrleitungssegmente, wird eine Steigerung der Strömungsgeschwindigkeit und, lokal auf den Krümmer begrenzt, des Druckverlustes je Meter Rohrleitung erreicht. Dies führt zu einer Homogenisierung der Strömung. Das Aufstauen von Flüssigkeit vor den Krümmern wird verhindert und so die Hauptursache für die Entstehung von Schwällen beseitigt.

Um eine ausreichende Steigerung der Geschwindigkeit zu erreichen, ist es bevorzugt, dass der Durchmesser der verbindenden Abschnitte kleiner oder gleich 0,95, bevorzugt 0,75 besonders bevorzugt 0,5 mal dem Durchmesser des Segments mit dem kleinsten Durchmesser ist.

Weiterhin wurde gefunden, dass sich ab einer gewissen Rohrlänge zwischen zwei Krümmern eine Schwallströmung direkt aus der vorliegenden Wellen- oder Schichtenströmung ausbildet. Um ein Entstehen von Schwällen in den vorwiegend horizontalen Segmenten zwischen den Krümmern sicher zu verhindern, sollte die Länge der Segmente kleiner als 250, bevorzugt kleiner als 100, besonders bevorzugt kleiner oder gleich 50 mal ihrem Durchmesser sein.
Bei der Verwendung von vorwiegend horizontalen Segmenten sollte deren Neigung im Bereich von -10° bis +10°, bevorzugt im Bereich -5° bis +5° und besonders bevorzugt im Bereich -3° bis +3° liegen. Dadurch kann gewährleistet werden, dass bei Reparaturen der Rohrreaktor vollständig entleert werden kann.
Das im Rohrreaktor strömende Medium ist zumeist ein Gemisch aus einer gas- und/oder dampfförmigen Phase und einer Flüssigkeit. Es ist jedoch prinzipiell auch möglich, dass der Anteil der Flüssigkeit durch Anheben des Systemdruckes bis hin zum Grenzfall der einphasigen Flüssigkeitsströmung gesteigert werden kann.

Falls erforderlich, kann der Rohrreaktor temperierbar ausgestaltet sein. Die Temperierung kann hierbei über eine Beheizung/Kühlung der Rohrwand oder innenliegende Wärmeübertragerflächen erfolgen.
Der Vorteil des erfindungsgemäß verwendeten Rohrreaktors besteht darin, dass zur Erreichung einer gleichmäßigen Strömung ohne Schwälle keinerlei Einbauten in den Rohren erforderlich sind. Hierdurch wird die Bildung von Belägen und Verstopfungen vermindert. Sollte dennoch eine Reinigung der Rohrleitung erforderlich sein, kann dies im Vergleich zu Bauformen, welche Einbauten beinhalten, viel leichter durchgeführt werden. Aufgrund der einfachen Ausführung können im Vergleich zu anderen Bauformen auch Investitionskosten eingespart werden.

Damit ist der erfindungsgemäß verwendete Rohrreaktor mit den oben näher beschriebenen konstruktiven Merkmalen auch für andere Reaktionen, in denen eine Gas- und eine Flüssigphase nebeneinander vorliegen, einsetzbar. Vorzugsweise ist der Durchmesser der verbindenden Abschnitte kleiner oder gleich 0,95, bevorzugt 0,75 besonders bevorzugt 0,5 mal dem Durchmesser des Segments mit dem kleinsten Durchmesser ist. Weiterhin ist bevorzugt, dass die Länge der Segmente kleiner als 250, bevorzugt kleiner als 100, besonders bevorzugt kleiner oder gleich 50 mal ihrem Durchmesser ist.

Bei dem erfindungsgemäßen Verfahren kann es zur Erreichung eines höheren Endumsatzes sinnvoll sein, dass der Austrag der des Rohrreaktors einer Destillationskolonne zur Nachreaktion zugeführt wird, in welcher die Flüssigphase von oben nach unten und die Gasphase von unten nach oben durch die Destillationskolonne geleitet wird.

Durch die Reduktion des Gesamtreaktionsvolumen lässt sich die Anlage wesentlich kompakter bauen. Dies stellt im Hinblick auf die Gefährlichkeit des in der Anlage gehandhabten Phosgens eine erhebliche Erhöhung der Sicherheit der Anlage und gleichzeitig eine Reduzierung der Investitionskosten dar.

Die Umsetzung des Amins mit dem Phosgen findet vorzugsweise bei Temperaturen von 60 bis 200°C und bei Absolutdrücken von 0,9 bar bis 400 bar statt, wobei das molare Verhältnis von Phosgen zu eingesetzten Aminogruppen insbesondere 15:1 bis 1:1 beträgt.

Die Verweilzeit des Reaktionsgemisches im Rohrreaktor beträgt je nach eingesetztem Amin von 5 s bis 3 h. Vorzugsweise liegt die Verweilzeit im Bereich von 5 s bis 1000 s, bevorzugt im Bereich von 20 s bis 500 s, besonders bevorzugt im Bereich von 25 s bis 200 s.

Die Vermischung der Reaktanden des erfindungsgemäßen Verfahrens a) wird vorzugsweise in einer Mischeinrichtung durchgeführt, die sich durch hohe turbulente Scherung des durch die Mischeinrichtung geführten Reaktionsgemischstromes auszeichnet, bevorzugt eine Rotationsmischeinrichtung, Mischpumpe oder eine Mischdüse, besonders bevorzugt eine Mischdüse, wobei die Mischeinrichtung dem Rohrreaktor vorangestellt ist. Derartige Düsen sind beispielsweise beschrieben in DD 300 168, WO 01/91898 und WO 02/02217.

Die Verweilzeit in Schritt b) ist zumeist ausreichend, um eine vollständige Umsetzung des Reaktionsgemisches zum Isocyanat zu gewährleisten. Dennoch kann es vorteilhaft sein, das aus dem Rohrreaktor austretende Reaktionsgemisch zur Vervollständigung der Umsetzung einem Nachreaktor, insbesondere einer Reaktionskolonne c) zuzuführen. Derartige Kolonnen werden beispielsweise in WO 99/54285 beschrieben.

In diesem Nachreaktor erfolgt die vollständige Zersetzung von noch im Reaktionsgemisch enthaltenem Carbamoylchlorid und Aminhydrochlorid. Die Bedingungen in dem Nachreaktor sind dabei so zu wählen, dass eine vollständige Umsetzung gewährleistet ist. Bei Verwendung einer Kolonne ist es vorteilhaft, wenn die Flüssigphase, wie Phosgen, Isocyanat und Lösungsmittel von oben nach unten und die Leichtsieder, wie Phosgen und Chlorwasserstoff, von unten nach oben geleitet werden.

Zwischen dem Rohrreaktor von Schritt b) und dem Nachreaktor von Schritt c) kann ein Abschnitt mit einem Durchmesser, wie ihn die verbindenden Abschnitte aufweisen, angebracht werden. Weiterhin kann es vorteilhaft sein, zwischen Rohrreaktor und Nachrektor eine Regelarmatur anzubringen, um einen gleichmäßigen Produkteingang in den Nachreaktor zu gewährleisten.

Für das erfindungsgemäße Verfahren kann ein beliebiges primäres Amin oder ein Gemisch aus zwei oder mehr dieser Amine eingesetzt werden. Beispiele sind Methylendiphenylamin (einzelne Isomere, Gemische von Isomeren und/oder Oligomeren), Toluylendiamin, n-Pentylamin, 6-Methyl-2-heptanamin, Cyclopentylamin, R,S-1-Phenylethylamin, 1-Methyl-3-phenylpropylamin, 2,6-Xylidin, 2-Dimethylaminoethylamin, 2-Diisopropylaminoethylamin, C11-Neodiamin, Isophorondiamin, 1,6-Hexamethylendiamin, Naphthyldiamin, 3,3'-Diaminodiphenylsulfon und 4-Aminomethyl-1,8-octandiamin. Bevorzugt werden aromatische Amine, insbesondere Toluylendiamin und Diaminodiphenylmethan oder dessen höhere Homologen eingesetzt.

Das erfindungsgemäße Verfahren eignet sich im allgemeinen zur Herstellung von beliebigen Isocyanaten. Besonders vorteilhaft kann das Verfahren zur Herstellung von Methylen(diphenylisocyanat) (MDI) und Toluylendiisocyanat (TDI) angewandt werden.

Bei dem erfindungsgemäßen Verfahren können die Einsatzkomponenten in einem inerten Lösungsmittel gelöst werden. Diese inerten Lösungsmittel sind üblicherweise organische Lösungsmittel, die einzeln oder im Gemische untereinander eingesetzt werden können. Dabei sind Chlorbenzol, Dichlorbenzol, Trichlorbenzol, Toluol, Hexan, Diethylisophthalat (DEIP), Tetrahydrofuran (THF), Dimethylformamid (DMF), Benzol und deren Gemische bevorzugt. Besonders bevorzugt ist Chlorbenzol. In einer besonderen Ausführungsform des erfindungsgemäßen Verfahrens kann als Lösungsmittel auch das im Verfahren hergestellte Isocyanat eingesetzt werden, wie beispielsweise in WO 96/16028 beschrieben.

Nach der Reaktion wird das Stoffgemisch bevorzugt mittels Rektifikation in Isocyanat(e), Lösungsmittel, Phosgen und Chlorwasserstoff aufgetrennt. Geringe Mengen von Nebenprodukten, die im Isocyanat verbleiben, können mittels zusätzlicher Rektifikaktion oder auch Kristallisation vom erwünschten Isocyanat getrennt werden.

Die nach dem erfindungsgemäßen Verfahren hergestellten Isocyanate können insbesondere zur Herstellung von Polyurethanen eingesetzt werden. Dazu werden sie in bekannter Weise mit Verbindungen mit mindestens zwei mit Isocyanatgruppen reaktiven Wasserstoffatomen umgesetzt.

Die Erfindung soll durch die nachstehenden Bespiele näher erläutert werden.

### Beispiele

In einer Versuchsapparatur wurde eine Rohrstrecke, die aus zwei über einen 180° Rohrkrümmer miteinander verbundenen, horizontal liegenden, geraden Rohren (DN 200, Länge jeweils 5 m) bestand, mit einem Flüssig-Gas-Gemisch durchströmt. Der Ausgang der Rohrstrecke lag dabei 1,2 m höher als der Eingang. Die Höhendifferenz wurde hauptsächlich in dem Rohrkrümmer überwunden. Als Rohrkrümmer wurden
a) ein Krümmer, dessen Durchmesser gleich zu dem Durchmessers der geraden Segmente des Reaktors ist (DN 200), und
b) ein Krümmer mit halbem Durchmesser der geraden Rohre (DN 100) verwendet.

In beiden Anordnungen wurde der Volumenstrom der Flüssigkeit im Bereich von 20 m³/h - 60 m³/h und der Volumenstrom des Gases im Bereich von 20 m³/h - 200 m³/h variiert. In jedem Versuch wurde die sich einstellende Strömungsform beobachtet, und die Druckdifferenz über den Rohrkrümmer gemessen.
a) Versuche in Anordnung mit Krümmer DN 200:
   Für alle Flüssigkeits- und Gasdurchsätze stellte sich fast ausschließlich Schwallströmung als Strömungsform im oberen Rohr ein. Diese wurde offensichtlich durch eine inhomogene Durchströmung des Rohrkrümmers verursacht. Der Druckabfall über den Krümmer betrug 0,2 bar bei 20 m³/h Flüssigkeit und 20 m³/h Gas, und stieg mit zunehmenden Volumenströmen bis 0,5 bar bei 60 m³/h Flüssigkeit und 200 m³/h Gas an.
b) Versuche in Anordnung mit Krümmer DN 100:
   Ab einem Volumenstrom der Flüssigkeit von 40 m³/h traten keine Flüssigkeitsschwälle mehr auf. Die beobachtete Strömungsform war eine Wellenströmung. Der Druckabfall über den Krümmer betrug 0,2 bar bei 20 m³/h Flüssigkeit und 20 m³/h Gas, und stieg mit zunehmenden Volumenströmen bis 0,6 bar bei 60 m³/h Flüssigkeit und 200 m³/h Gas an. Der im Vergleich zum Fall a) höhere Druckabfall trug somit zu einer Vermischung der Phasen im Krümmer und damit zu einer Homogenisierung der Strömung bei.

## Patentansprüche

1. Verfahren zur Herstellung von Polyisocyanaten durch Umsetzung von primären Aminen mit Phosgen, umfassend die Schritte
a) Mischung des Amins mir dem Phosgen
b) Umsetzung des Amins mit dem Phosgen in einem Verweilzeitreaktor, gegebenenfalls
c) Überführung des Austrags des Reaktors aus Schritt b) in eine Destillationskolonne,
**dadurch gekennzeichnet, dass** der Verweilzeitreaktor in Schritt b) als Rohrreaktor ausgestaltet ist, der aus mehreren horizontalen und/oder vertikalen Segmenten besteht, die untereinander durch Abschnitte verbunden sind, wobei die verbindenden Abschnitte einen Durchmesser aufweisen, der geringer ist als der Durchmesser des engsten horizontalen oder vertikalen Segments.

2. Verfahren nach Anspruch 1, **dadurch gekennzeichnet, dass** die verbindenden Abschnitte als Rohrleitungskrümmer ausgestaltet sind.

3. Verfahren nach Anspruch 1, **dadurch gekennzeichnet, dass** der Durchmesser der verbindenden Abschnitte kleiner oder gleich einem Wert von 0,95 mal dem kleinsten Durchmesser der horizontalen und/oder vertikalen Segmente ist.

4. Verfahren nach Anspruch 1, **dadurch gekennzeichnet, dass** der Durchmesser der verbindenden Abschnitte kleiner oder gleich einem Wert 0,75 mal dem kleinsten Durchmesser der horizontalen und/oder vertikalen Segmente ist.

5. Verfahren nach Anspruch 1, **dadurch gekennzeichnet, dass** der Durchmesser der verbindenden Abschnitte kleiner oder gleich einem Wert von 0,5 mal dem kleinsten Durchmesser der horizontalen und/oder vertikalen Segmente ist.

6. Verfahren nach einem der vorhergehenden Ansprüche, **dadurch gekennzeichnet, dass** die Länge der horizontalen und/oder vertikalen Segmente kleiner als 250, bevorzugt kleiner als 100, besonders bevorzugt kleiner oder gleich 50 mal dem Durchmesser der Rohrleitung sein.

7. Verfahren nach einem der vorhergehenden Ansprüche, **dadurch gekennzeichnet, dass** das strömende Medium ein Gemisch aus einer gas- und/oder dampfförmigen Phase und einer Flüssigkeit ist.

8. Verfahren nach einem der vorhergehenden Ansprüche, **dadurch gekennzeichnet, dass** der Anteil der Flüssigkeit durch Anheben des Systemdruckes bis hin zum Grenzfall der einphasigen Flüssigkeitsströmung gesteigert werden kann.

9. Verfahren nach einem der vorhergehenden Ansprüche, **dadurch gekennzeichnet, dass** die Verweilzeit der Flüssigkeit in Schritt b) im Bereich von 5 s bis 1000 s liegt.

10. Verfahren nach einem der vorhergehenden Ansprüche, **dadurch gekennzeichnet, dass** die Verweilzeit der Flüssigkeit in Schritt b) im Bereich von 20 s bis 500 s liegt.

11. Verfahren nach einem der vorhergehenden Ansprüche, **dadurch gekennzeichnet, dass** die Verweilzeit der Flüssigkeit in Schritt b) im Bereich von 25 s bis 200 s liegt.

12. Verfahren nach einem der vorhergehenden Ansprüche, **dadurch gekennzeichnet, dass** die horizontalen Segmente eine Neigung gegen die Horizontale im Bereich von -10° bis +10°, bevorzugt im Bereich -5° bis +5° und besonders bevorzugt im Bereich -3° bis +3° aufweisen.

13. Verfahren gemäß einem der vorhergehenden Ansprüche, **dadurch gekennzeichnet, dass** das Amin ausgewählt ist aus der Gruppe, enthaltend Methylendiphenylamin, dessen höhere Homologe, Toluylendiamin, n-Pentylamin, 6-Methyl-2-heptanamin, Cyclopentylamin, R,S-1-Phenylethylamin, 1-Methyl-3-phenylpropylamin, 2,6-Xylidin, 2-Dimethylaminoethylamin, 2-Diisopropylaminoethylamin, C11-Neodiamin, Isophorondiamin, 1,6-Hexamethylnediamin, Naphthyldiamin, 3,3'-Diaminodiphenylsulfon und 4-Aminomethyl-1,8-octandiamin.

14. Verwendung eines Rohrreaktors, der aus mehreren horizontalen und/oder vertikalen Segmenten besteht, die untereinander durch Abschnitte verbunden sind, wobei die verbindenden Abschnitte einen Durchmesser aufweisen, der geringer ist als der Durchmesser des engsten horizontalen oder vertikalen Segments zur Herstellung von Isocyanaten durch Umsetzung von primären Aminen mit Phosgen.

## Claims

1. A process for preparing polyisocyanates by reacting primary amines with phosgene, which comprises the steps
a) mixing the amine with the phosgene,
b) reacting the amine with the phosgene in a residence reactor and, if appropriate,
c) transferring the output from the reactor of step b) into a distillation column,
wherein the residence reactor in step b) is configured as a tube reactor which comprises a plurality of horizontal and/or vertical segments which are connected to one another by sections which have a diameter less than the diameter of the narrowest horizontal or vertical segment.

2. The process according to claim 1, wherein the connecting sections are configured as pipe bends.

3. The process according to claim 1, wherein the diameter of the connecting sections is less than or equal to a value of 0.95 times the smallest diameter of the horizontal and/or vertical segments.

4. The process according to claim 1, wherein the diameter of the connecting sections is less than or equal to a value of 0.75 times the smallest diameter of the horizontal and/or vertical segments.

5. The process according to claim 1, wherein the diameter of the connecting sections is less than or equal to a value of 0.5 times the smallest diameter of the horizontal and/or vertical segments.

6. The process according to any of the preceding claims, wherein the length of the horizontal and/or vertical segments is less than 250 times, preferably less than 100 times and particularly preferably less than or equal to 50 times, the diameter of the tube.

7. The process according to any of the preceding claims, wherein the flowing medium is a mixture of a gaseous and/or vapor phase and a liquid.

8. The process according to any of the preceding claims, wherein the proportion of liquid can be increased by increasing the system pressure up to the limiting case of single-phase liquid flow.

9. The process according to any of the preceding claims, wherein the residence time of the liquid in step b) is in the range from 5 s to 1000 s.

10. The process according to any of the preceding claims, wherein the residence time of the liquid in step b) is in the range from 20 s to 500 s.

11. The process according to any of the preceding claims, wherein the residence time of the liquid in step b) is in the range from 25 s to 200 s.

12. The process according to any of the preceding claims, wherein the horizontal segments have an inclination to the horizontal in the range from -10° to +10°, preferably in the range from -5° to +5° and particularly preferably in the range from -3° to +3°.

13. The process according to any of the preceding claims, wherein the amine is selected from the group consisting of methylenedi(phenylamine), its higher homologues, toluenediamine, n-pentylamine, 6-methyl-2-heptanamine, cyclopentylamine, R,S-1-phenylethylamine, 1-methyl-3-phenylpropylamine, 2,6-xylidine, 2-dimethylaminoethylamine, 2-diisopropylaminoethylamine, C11-neodiamine, iso-phoronediamine, 1,6-hexamethylenediamine, naphthylenediamine, bis(3,3'-aminophenyl) sulfone and 4-aminomethyl-1,8-octanediamine.

14. The use of a tube reactor comprising a plurality of horizontal and/or vertical segments which are connected to one another by sections which have a diameter less than the diameter of the narrowest horizontal or vertical segment for preparing isocyanates by reacting primary amines with phosgene.

## Revendications

1. Procédé pour la préparation de polyisocyanates par transformation d'amines primaires avec du phosgène, comprenant les étapes
a) mélange de l'amine avec le phosgène
b) transformation de l'amine avec le phosgène dans un réacteur à durée de séjour, le cas échéant
c) transfert du produit évacué du réacteur de l'étape b) dans une colonne de distillation,
**caractérisé en ce que** le réacteur à durée de séjour dans l'étape b) est réalisé comme réacteur tubulaire, qui est constitué par plusieurs segments horizontaux et/ou verticaux, qui sont reliés l'un à l'autre par des sections, les sections de liaison présentant un diamètre qui est inférieur au diamètre du segment horizontal ou vertical le plus étroit.

2. Procédé selon la revendication 1, **caractérisé en ce que** les sections de liaison sont réalisées sous forme de coudes de conduite tubulaire.

3. Procédé selon la revendication 1, **caractérisé en ce que** le diamètre des sections de liaison est inférieur ou égal à une valeur représentant 0,95 fois le diamètre le plus petit des segments horizontaux et/ou verticaux.

4. Procédé selon la revendication 1, **caractérisé en ce que** le diamètre des sections de liaison est inférieur ou égal à une valeur représentant 0,75 fois le diamètre le plus petit des segments horizontaux et/ou verticaux.

5. Procédé selon la revendication 1, **caractérisé en ce que** le diamètre des sections de liaison est inférieur ou égal à une valeur représentant 0,5 fois le diamètre le plus petit des segments horizontaux et/ou verticaux.

6. Procédé selon l'une quelconque des revendications précédentes, **caractérisé en ce que** la longueur des segments horizontaux et/ou verticaux est inférieure à 250, de préférence inférieure à 100, de manière particulièrement préférée inférieure ou égale à 50 fois le diamètre de la conduite tubulaire.

7. Procédé selon l'une quelconque des revendications précédentes, **caractérisé en ce que** l'agent en écoulement est un mélange d'une phase sous forme de gaz et/ou de vapeur et d'un liquide.

8. Procédé selon l'une quelconque des revendications précédentes, **caractérisé en ce que** la proportion de liquide peut être augmentée, par l'augmentation de la pression du système, jusqu'au cas limite d'un flux de liquide à une phase.

9. Procédé selon l'une quelconque des revendications précédentes, **caractérisé en ce que** le temps de séjour du liquide dans l'étape b) est situé dans la plage de 5 s à 1000 s.

10. Procédé selon l'une quelconque des revendications précédentes, **caractérisé en ce que** le temps de séjour du liquide dans l'étape b) est situé dans la plage de 20 s à 500 s.

11. Procédé selon l'une quelconque des revendications précédentes, **caractérisé en ce que** le temps de séjour du liquide dans l'étape b) est situé dans la plage de 25 s à 200 s.

12. Procédé selon l'une quelconque des revendications précédentes, **caractérisé en ce que** les segments horizontaux présentent une pente par rapport à l'horizontale dans la plage de -10° à +10°, de préférence dans la plage de -5° à +5° et de manière particulièrement préférée dans la plage de -3° à +3°.

13. Procédé selon l'une quelconque des revendications précédentes, **caractérisé en ce que** l'amine est choisie dans le groupe contenant la méthylènediphénylamine, ses homologues supérieurs, la toluylènediamine, la n-pentylamine, la 6-méthyl-2-heptanamine, la cyclopentylamine, la R,S-1-phényléthylamine, la 1-méthyl-3-phénylpropylamine, la 2,6-xylidine, la 2-diméthylaminoéthylamine, la 2-diisopropylaminoéthylamine, la C₁₁-néodiamine, l'isophoronediamine, la 1,6-hexaméthylènediamine, la naphtyldiamine, la 3,3'-diaminodiphénylsulfone et la 4-aminométhyl-1,8-octanediamine.

14. Utilisation d'un réacteur tubulaire, qui est constitué par plusieurs segments horizontaux et/ou verticaux, qui sont reliés l'un à l'autre par des sections, les sections de liaison présentant un diamètre qui est inférieur au diamètre du segment horizontal ou vertical le plus étroit pour la préparation d'isocyanates par transformation d'amines primaires avec du phosgène.
